(19) **Europäisches Patentamt**

**European Patent Office**

**Office européen des brevets**

(11) Veröffentlichungsnummer: **0 043 983**
**B1**

(12) **EUROPÄISCHE PATENTSCHRIFT**

(45) Veröffentlichungstag der Patentschrift:
**12.03.86**

(51) Int. Cl.⁴: **A 61 G 3/00**

(21) Anmeldenummer: **81105082.2**

(22) Anmeldetag: **01.07.81**

(54) **Krankentrage.**

(30) Priorität: **11.07.80 DE 3026406**
**06.11.80 DE 3041932**
**22.06.81 DE 3124416**

(43) Veröffentlichungstag der Anmeldung:
**20.01.82 Patentblatt 82/3**

(45) Bekanntmachung des Hinweises auf die Patenterteilung:
**12.03.86 Patentblatt 86/11**

(84) Benannte Vertragsstaaten:
**BE FR GB IT LU NL**

(56) Entgegenhaltungen:
**DE - C - 129 622**
**FR - A - 2 437 824**
**US - A - 3 204 256**
**US - A - 3 732 863**
**US - A - 4 124 908**
**US - A - 4 204 529**

(73) Patentinhaber: **Christian Miesen Fahrzeug- und Karosseriewerk GmbH,
Dottendorfer-/Christian-Miesen-Strasse, D-5300 Bonn (DE)**

(72) Erfinder: **Schnitzler, Alois, Hummerich Bitze 9,
D-5300 Bonn 3 (DE)**

(74) Vertreter: **Brose, Karl A. et al, Wiener Strasse 2,
D-8023 München-Pullach (DE)**

ACTORUM AG

## Beschreibung

Die Erfindung betrifft eine Krankentrage mit einem verstellbaren und arretierbaren Gurt, der als Sicherheitsgurt zur Halterung eines auf der Krankentrage liegenden Patienten dient.

Ferner betrifft die Erfindung ein Rückhaltekissen, mit dem eine herkömmliche Krankentrage zu einer erfindungsgemässen nachgerüstet werden kann.

Beim Transport von Patienten auf Krankentragen in einem Krankenwagen kann es gelegentlich zu stossartigen Bewegungsabläufen, insbesondere Verzögerungen kommen. Dies trifft insbesondere dann zu, wenn der Krankenwagen bei einem Unfall auf ein Hindernis auffährt. Um in einem derartigen Fall eine zusätzliche Verletzung des Patienten zu verhindern, wurde bereits ein sogenannter Schulterschräggurt für eine Krankentrage entwickelt (vgl. DE-C-2 543 473), der schräg über eine Schulter eines Patienten geführt ist. Dieser Schulterschräggurt ist am Kopfende der Krankentrage angebracht, so dass die Schulter des betreffenden Patienten wenigstens 20 cm vom Kopfende der Krankentrage entfernt ist. Diese Entfernung von 20 cm ist dabei sogar ein unterer Grenzwert für das Mass von der Schulter eines Patienten bis zu dessen Kopfende. Sollte ein Krankenwagen mit einer Krankentrage, auf der ein Patient mit einem derartigen Schulterschräggurt gesichert ist, in einen Auffahrunfall verwickelt werden, so rutscht der Patient zunächst diese Entfernung von ca. 20 cm nach vorne und findet erst dann Halt im Sicherheitsgurt; gegebenenfalls rutscht der Patient sogar mit seinem Kopf über das Kopfende der Krankentrage hinaus, so dass sein Kopf nach unten fallen kann.

Um derartige Nachteile zu vermeiden, wurde vom Erfinder bereits daran gedacht, den Sicherheitsgurt in Schulterhöhe anzubringen, was jedoch gewisse Nachteile hinsichtlich einer Einengung der Liegefläche für den Patienten mit sich bringen könnte; auch wären zusätzliche Verletzungen infolge einer derartigen Einengung nicht unbedingt auszuschliessen. Weiterhin könnte der Sicherheitsgurt auch unterhalb der Krankentrage angebracht werden, also nicht an deren Kopfende. Hierzu müsste jedoch der Sicherheitsgurt durch den Krankentragenbezug und deren Auflage hindurchgeführt werden. Da die Auflage jedoch abnehmbar sein sollte, wäre es umständlich und zeitraubend, den Sicherheitsgurt ständig erneut in den Tragenbezug und die Auflage einzuführen. Zudem müssten die Auflage und der Tragenbezug mit entsprechenden Öffnungen versehen werden.

Es ist daher Aufgabe der Erfindung, eine Krankentrage der eingangs genannten Art so weiterzubilden, dass Verletzungen des Patienten auch bei stossartigen Bewegungen in Längsrichtung der Krankentrage ausgeschlossen sind, dass der Patient demnach auch bei stossartigen Bewegungen in Längsrichtung der Krankentrage sicher auf der Krankentrage gehalten wird. Diese Aufgabe wird erfindungsgemäss dadurch gelöst, dass an der Krankentrage ein Rückhaltekissen gehalten ist, das über eine Rückhaltefläche an der Schulter des Patienten anliegt, so dass dieser bezüglich der Krankentrage auch bei stossartigen Bewegungsabläufen in deren Längsrichtung ortsfest bleibt.

Ferner wird diese Aufgabe durch ein Rückhaltekissen mit den in Anspruch 37 angegebenen Merkmalen gelöst, mit dem eine herkömmliche Krankentrage zu einer erfindungsgemässen nachrüstbar ist.

Vorzugsweise ist eine Auswölbung im Rückhaltekissen für Kopf und Hals des Patienten vorgesehen.

Ausserdem ist in vorteilhafter Weise das Rückhaltekissen mit dem Sicherheitsgurt integriert, wobei ein weiterer Sicherheitsgurt vorgesehen sein kann und der Sicherheitsgurt und der weitere Sicherheitsgurt jeweils über eine Schulter des Patienten geführt sind. Der Sicherheitsgurt und der weitere Sicherheitsgurt sind dabei mit ihrem einen Ende am Rückhaltekissen und mit ihrem anderen Ende an Seitenholmen des Kopfendes der Krankentrage befestigt. Das andere Ende des Sicherheitsgurtes und des weiteren Sicherheitsgurtes kann dabei an den Seitenholmen in Höhe eines quer zur Längsrichtung der Krankentrage verlaufenden Befestigungsgurtes angebracht sein. Ausserdem ist es möglich, dass der Sicherheitsgurt und der weitere Sicherheitsgurt direkt am Befestigungsgurt angebracht sind.

In vorteilhafter Weise wird wenigstens ein Gurt zum Befestigen des Rückhaltekissens an der Krankentrage verwendet. Der Gurt und gegebenenfalls ein weiterer Gurt sind dabei jeweils an einem Seitenholm des Kopfendes der Krankentrage befestigbar.

Der Sicherheitsgurt und gegebenenfalls der weitere Sicherheitsgurt können über ein Automatikgehäuse am Rückhaltekissen angebracht werden. Schliesslich ist es noch vorteilhaft, dass die Sicherheitsgurte und die zum Befestigen des Rückhaltekissens dienenden Gurte in ihrer Länge verstellbar sind.

Um einerseits ein abruptes Abfangen des Patienten in einer Gefahrensituation zu vermeiden und andererseits die Auflagematratze der Krankentrage leicht mit einem Bezug überziehen zu können, ist es gemäss einer alternativen Ausführungsform bevorzugt, dass das Rückhaltekissen an der Krankentrage elastisch gelagert ist, so dass ein abruptes Abfangen des Patienten bei den stossartigen Bewegungsabläufen vermieden wird. Durch eine solche elastische Lagerung des Rückhaltekissens können die bei einem Aufprall des Patienten auftretenden Trägheitskräfte schonend abgefangen werden; ausserdem bietet die erfindungsgemässe Krankentrage eine wesentliche Arbeitserleichterung für den Sanitäter, da zum Überziehen der Auflagematratze mit einer Decke oder einem Papierüberzug nicht zuerst das Rückhaltekissen entfernt werden muss. Schliesslich kann das Rückhaltekissen auch ohne grössere Umbauten bei bestehenden Krankentragen oder sonstigen Fahrzeugen angebracht werden.

Bei dieser Ausführungsform der Erfindung ist also das Rückhaltekissen nicht mit einem Sicherheitsgurt integriert; es wird vielmehr elastisch gelagert, wozu beispielsweise eine Feder dienen kann. Diese Feder kann ihrerseits auf einem Gestänge angeordnet werden. Das Gestänge mit der Feder kann in vorteilhafter Weise mit dem Rückhaltekissen integriert sein.

Vorzugsweise ist das Gestänge am Kopfende der Krankentrage verankerbar. Es weist zwei in Längsrichtung der Krankentrage verlaufende Kolbenstangen auf, auf denen jeweils ein Kolbengehäuse mit einer Feder gleitet. Die beiden Kolbengehäuse können starr durch eine Platte miteinander verbunden sein.

Das Gestänge bzw. der Federmechanismus befindet sich unterhalb des eigentlichen Kissens. Dabei kann das Gestänge an der Unterseite einer mit Verstärkungslaschen verstärkten Holzplatte angebracht sein, auf deren Oberseite sich ein gegossenes Hartschaum-Formteil befindet, das im Kopfbereich mit Weichschaum belegt ist. Das Hartschaum-Formteil ist zusammen mit dem Weichschaum mit einem Kunstlederbezug versehen, der am unteren Rand des Brettes befestigt wird.

Ein Polstervorbau des Rückhaltekissens verhindert, dass der Körper eines Patienten über das Rückhaltekissen hinausrutscht, das infolge der Beweglichkeit des Rückhaltekissens über die Hublänge der Kolbenstangen keine Sicherheitsgurte verwendet werden müssen.

Eine noch grössere Sicherheit gegen Verletzungen eines auf einer derartigen Krankentrage transportierten Patienten kann dadurch erreicht werden, dass das Rückhaltekissen an einem Haltegurt befestigt ist, dass ein Sicherheitsgurt mit Aufrollautomatik und einem Kupplungsteil vorgesehen ist, dass am Fahrzeugboden ein entsprechendes zweites Kupplungsteil vorgesehen ist, in welchen sich der Kupplungsteil des Sicherheitsgurtes beim Einladen der Krankentrage in eine Trageneinrichtung selbsttätig einhängt und dass der Haltegurt mit der Aufrollautomatik derart verbunden ist, dass eine Zugkraft in dem Haltegurt die Aufrollautomatik sperrt.

Hierdurch wird insbesondere der Vorteil erzielt, dass bei Überschreiten eines bestimmten Verzögerungswertes oder wenn sich die Krankentrage aus ihrer Verankerung löst, der Patient immer noch am Fahrzeugboden mittelbar über die Krankentrage gehaltert wird. Ein weiterer Vorteil besteht darin, dass der Sicherheitsgurt mit seiner Kupplung beim Beladen der Trage mit dem Fahrzeugboden lösbar verbunden wird. Der mit dem Aufrollautomaten und dem Rückhaltekissen verbundene Haltegurt zieht bei einer ruckartigen Bewegung des Rückhaltekissens nach vorne die Sperre des Aufrollautomaten an, so dass der mit dem Fahrzeugboden verbundene Sicherheitsgurt gesperrt wird und der Patient mit der Krankentrage am Fahrzeugboden festgehalten wird. Ein besonderer Vorteil der oben geschilderten Lösung besteht noch darin, dass bei Einrichtungen, bei denen die Krankentrage vorgezogen oder in Kopftieflage gebracht werden kann, der Aufrollautomat derartige normale Bewegungen ermöglicht und nur bei plötzlichen Veränderungen, beispielsweise beim Bremsen oder bei Unfall, sperrt.

Im einzelnen kann die Erfindung dadurch weitergebildet werden, dass die Aufrollautomatik an einem Querträger der Krankentrage im Bereich des Fussendes angeordnet ist. Hierdurch wird erreicht, dass beim Beladen leicht und bequem das sichere Kuppeln des Sicherheitsgurtes mit dem Fahrzeugboden kontrolliert werden kann.

Eine besonders bevorzugte Ausführungsform nach der Erfindung wird dadurch geschaffen, dass der Haltegurt unterhalb der Liegefläche der Krankentrage verlaufend angeordnet ist.

Im einzelnen ist es vorteilhaft, dass der Haltegurt im Bereich des Kopfendes der Krankentrage seitlich gegabelt ausgebildet ist und an beiden Seiten des Rückhaltekissens Befestigungen aufweist. Hierdurch wird eine besonders stabile Halterung des Rückhaltekissens gewährleistet.

Die Befestigungen sind bevorzugt lösbar ausgebildet, um beispielsweise Bezüge od. dgl. leicht auswechseln zu können.

Eine besonders bevorzugte Ausführungsform nach der Erfindung kann dadurch geschaffen werden, dass der Kupplungsteil des Sicherheitsgurtes als Halteschlaufe ausgebildet ist und dass der Kupplungsteil am Fahrzeugboden als Haken od. dgl. ausgebildet ist, in welchen sich die Halteschlaufe selbsttätig einhängt.

Hierzu ist es von besonderem Vorteil, die aufgerollte Länge des Sicherheitsgurtes derart zu begrenzen, dass im eingerollten Zustand die Halteschlaufe sich in geringem Abstand über dem Fahrzeugboden befindet. Durch diese besondere Ausführungsform wird ein selbsttätiges Einklinken des Sicherheitsgurtes auf einfachste Weise erreicht.

Eine Ausführungsform nach der Erfindung kann dadurch geschaffen werden, dass der Haltegurt direkt mit der Sperre des Aufrollautomaten verbunden ist.

Alternativ hierzu ist es vorteilhaft, dass der Haltegurt im Bereich des Fussendes der Krankentrage nach oben und unten gegabelt ausgebildet ist, wobei ein Abschnitt der Gabelung mit der Sperre verbunden ist und der andere Abschnitt die Zugkräfte aufnimmt. Eine demgegenüber besonders bevorzugte Ausführungsform wird dadurch geschaffen, dass der Haltegurt im Bereich des Fussendes der Krankentrage nach oben und unten gegabelt ausgebildet ist, dass die beiden Abschnitte der Gabelung mit dem Gehäuse der Aufrollautomatik fest verbunden sind und dass einer der Abschnitte derart angeordnet ist, dass eine Winkelveränderung zwischen den Abschnitten die Sperre der Aufrollautomatik auslöst.

Die Vorteile nach der Erfindung werden ebenfalls bei der Verwendung der geschilderten Merkmale bei Liege-Tragsesseln erzielt.

Weitere vorteilhafte Einzelheiten der Erfindung sind den übrigen Unteransprüchen zu entnehmen.

Nachfolgend werden mehrere Ausführungsbeispiele der Erfindung anhand der Zeichnungen näher erläutert. In den Zeichnung zeigen:

Figur 1 ein erstes Ausführungsbeispiel einer erfindungsgemässen Krankentrage in Perspektive;

Figur 2 das Rückhaltekissen der Krankentrage der Fig. 1 mit zusätzlichen Einzelheiten;

Figur 3 ein weiteres Ausführungsbeispiel einer Krankentrage, auf der das Rückhaltekissen gemäss Fig. 4 und 5 anbringbar ist;

Figur 4 und Figur 5 das Rückhaltekissen für die Krankentrage gemäss Fig. 3 im entspannten bzw. gespannten Zustand;

Figur 6 eine Seitenansicht des Rückhaltekissens gemäss Fig. 4 und 5 mit einem Polstervorbau;

Figur 7 eine schematische perspektivische Darstellung der konstruktiven Einzelheiten einer weiteren Ausführungsform nach der Erfindung;

Figur 8 eine schematische Darstellung des Funktionsprinzips der Ausführungsform gemäss Figur 7 im Ruhezustand und

Figur 9 eine Ansicht gemäss Figur 8, jedoch beim Bremsen bzw. einem Unfall des Krankenfahrzeuges.

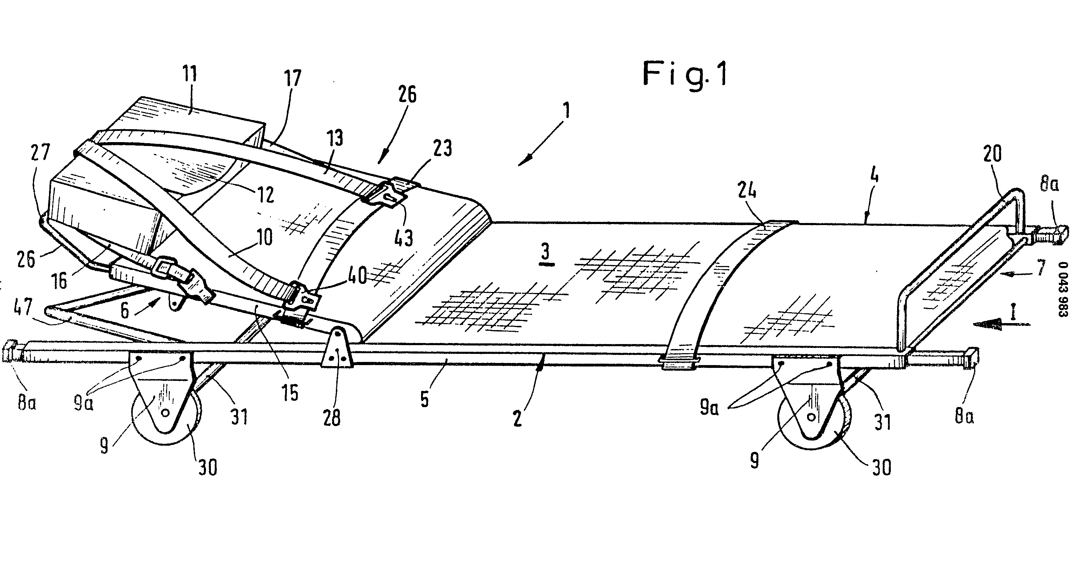

Figur 1 zeigt ein Ausführungsbeispiel einer Krankentrage 1, wie sie vorzugsweise in Krankenwagen verwendet wird. Die Krankentrage 1 besteht aus einem Traggestell 2, zwischen dem sich eine Auflagefläche frei erstreckt, auf die ein nicht dargestellter Patient zu liegen kommt. Die Auflagefläche 3 besteht im allgemeinen aus einem Tuch, das an dem Traggestell befestigt ist. Das Traggestell 2 selbst besteht aus Längsholmen 4, 5, die aus zylindrischen Hohlrohren gebildet sind. Die Hohlrohre können einen unterschiedlichen Querschnitt aufweisen. Am Kopfende 6 und am Fussende 7 der Krankentrage 1 sind in den Längsholmen 4, 5 ausziehbare Tragegriffe aufgenommen, die an ihren Enden kopfartige Abschlussstücke 8a tragen, die ein zu tiefes Hineinschieben der Tragegriffe in die Längsholme 4, 5 vermeiden. Die ausziehbaren Tragegriffe sind ausserdem mit nicht dargestellten Anschlägen versehen, die verhindern, dass Tragegriffe aus den Längsholmen 4, 5 vollständig herausgezogen werden können. An den Längsholmen 4, 5 sind weiterhin im Bereich des Kopfendes 6 und des Fussendes 7 der Krankentrage 1 nach unten abstehende Halterungsarme 9 über Schrauben oder Nieten 9a befestigt oder angeschweisst. An den unteren Enden der Halterungsarme 9 befinden sich Räder 30. Die Räder 30 sind im dargestellten Fall auf Achsen 31 befestigt, die sich zwischen den Paaren der Halterungsarme 9 am Kopfende 6 und am Fussende 7 erstrecken und in diesen gehaltert sind.

Die in Figur 1 dargestellte Krankentrage 1 enthält ausserdem eine nach oben klappbare Rükkenlehne 26, die aus zwei an ihren Enden mit einem Bügel 27 verbundenen Seitenholmen 15 (lediglich ein Seitenholm 15 ist gezeigt) besteht, zwischen dem sich ein Teil der Auflagefläche 3 erstreckt. Die unteren Enden der Seitenholme 15 sind schwenkbar in Verbindungsgliedern 28 gehaltert, die ihrerseits an den Längsholmen 4 und 5 befestigt sind. Zwischen den Längsholmen 4 und 5 erstrecken sich ausserdem ein Bügel 20, der eine Fussstütze für den sich auf der Krankentrage 1 befindlichen Patienten bildet, und ein Befestigungsgurt 24, der zum Festlegen der Beine des Patienten dient. Ausserdem ist noch zwischen den Holmen 15 ein Befestigungsgurt 23 vorgesehen, der den Patienten im Bereich seines Oberkörpers hält.

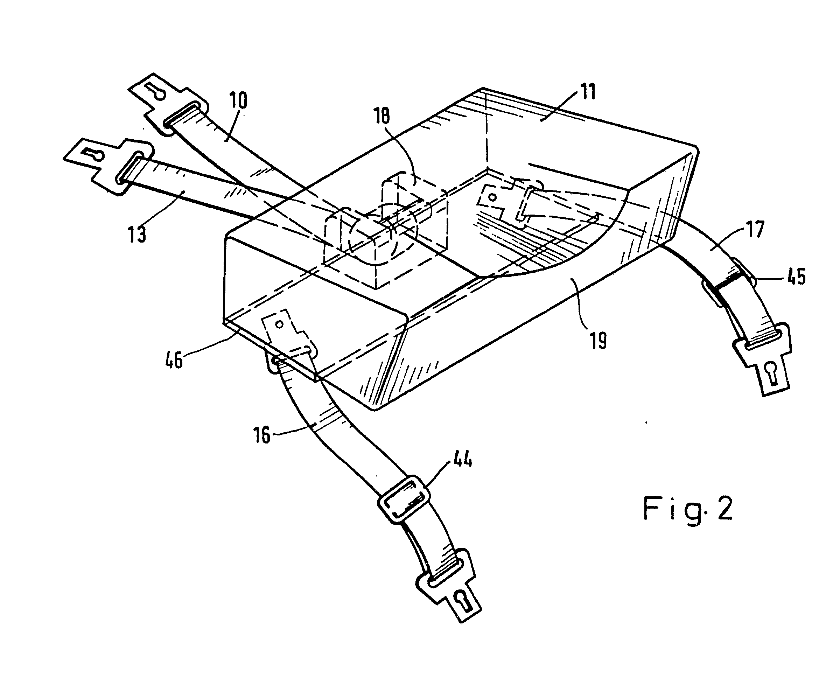

Beim dargestellten Ausführungsbeispiel der Erfindung sind zwei Sicherheitsgurte 10, 13 über Befestigungseinrichtungen 40, 43, die vorzugsweise als Schnelleinrastverbindungen ausgeführt sind, jeweils über eine Schulter des Patienten zur Unterseite eines Rückhaltekissens 11 führt. Am Rückhaltekissen 11 sind dabei die Sicherheitsgurte 10, 13 über ein Automatikgehäuse 18 (vgl. Figur 2) befestigt. Das Sicherheitskissen 11 enthält eine Auswölbung 12, in die Kopf und Hals des Patienten aufgenommen werden können. Wenn sich ein Patient auf der Krankentrage 1 befindet, so liegen seine Schultern an der Rückhaltefläche 19 (vgl. Fig. 2) des Rückhaltekissens 11 an, wobei die Sicherheitsgurte 10, 13 jeweils über eine Schulter dieses Patienten geführt sind. Der Befestigungsgurt 23 ist ausserdem über den Oberkörper des Patienten geführt. Die Verbindungseinrichtungen 40, 43 können dabei an diesem Befestigungsgurt 23 vorgesehen sein. Ausserdem sind Gurte 16, 17 vorgesehen, die wie die Gurte 10, 13 über Schnallen 44, 45 in ihrer Länge einstellbar sind. Diese Gurte 16, 17 sind an einer Unterlage 46 des Rückhaltekissens 11 angebracht, an der auch das Automatikgehäuse 18 festgelegt ist. Diese Unterlage 46 ist im Gegensatz zum Rückhaltekissen 11 starr. Weiterhin sind die Gurte 16, 17 an den Holmen 15 der Rückenlehne 26 befestigbar, die über ein Gestänge 47 höhenverstellbar ist.

Bei einer stossartigen Verzögerung des Bewegungsablaufes der Krankentrage 1 in Richtung des Pfeiles 2' nimmt die Rückhaltefläche 19 des Rückhaltekissens 11 die durch den Patienten ausgeübte Trägheitskraft mittels der Sicherheitsgurte 10, 13 auf, so dass sich der Patient bezüglich der Krankentrage 1 ortsfest verhält. Dadurch werden Verletzungen des Patienten durch stossartige Bewegungsabläufe der Krankentrage 1 weitgehend vermieden.

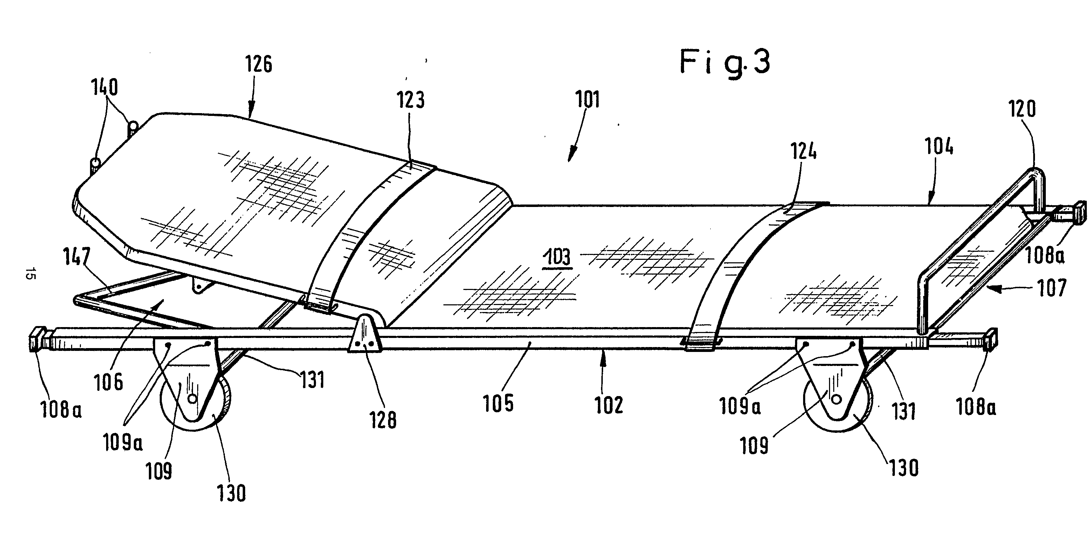

Die in der Fig. 3 dargestellte Krankentrage 101 besteht aus einem Traggestell 102, zwischen dem sich eine Auflagefläche 103 frei erstreckt, auf die ein nicht dargestellter Patient zum Liegen kommt. Die Auflagefläche 103 besteht im allgemeinen aus einem Tuch, das an dem Traggestell 102 befestigt ist. Das Traggestell 102 selbst weist Längsholme 104, 105 auf, die aus zylindrischen Hohlrohren gebildet sind. Die Hohlrohre können einen unterschiedlichen Querschnitt haben. Am Kopfende 106 und am Fussende 107 der Krankentrage 101 sind in den Längsholmen 104, 105 ausziehbare Tragegriffe aufgenommen, die an ihren Enden kopfartige Abschlussstücke 108a tragen, die ein zu tiefes Hineinschieben der Tragegriffe in die Längsholme 104, 105 vermeiden. Die ausziehbaren Tragegriffe sind ausserdem mit nicht dargestellten Anschlägen versehen, die verhindern, dass die Tragegriffe aus den Längsholmen 104, 105 vollständig herausgezogen werden können. An den Längsholmen 104, 105 sind weiterhin im Bereich des Kopfendes 106 und des Fussendes 107 der Krankentrage 101 nach unten abstehende Halterungsarme 109 über Schrauben oder Nieten 109a befestigt oder angeschweisst. An den unteren Enden der Halterungsarme 109 befinden sich Räder 130. Die Räder 130 sind im dargestellten Ausführungsbeispiel auf Achsen 131 befestigt, die sich zwischen den Paaren der Halterungsarme 109 am Kopfende 106 und am Fussende 107 erstrecken und in diesen gehaltert sind.

Die in der Fig. 3 dargestellte Krankentrage hat ausserdem eine nach oben klappbare Rückenlehne 126, die an ihrem oberen Ende 102 Halterungen 140 besitzt, in die Rohre einschiebbar sind. Die Rückenlehne 126 ist schwenkbar in Verbindungs-gliedern 128 gehalten, die ihrerseits an den Längsholmen 104 und 105 befestigt sind. Zwischen den Längsholmen 104, 105 erstrecken sich ausserdem ein Bügel 120, der eine Fussstütze für den sich auf der Krankentrage 101 befindlichen Patienten bildet, und ein Befestigungsgurt 124, der zum Festlegen der Beine des Patienten dient. Ausserdem ist noch an der Rückenlehne 126 ein Befestigungsgurt 123 vorgesehen, der den Patienten im Bereich seines Oberkörpers hält. Die Rückenlehne 126 selbst ist über ein Gestänge 147 höhenverstellbar.

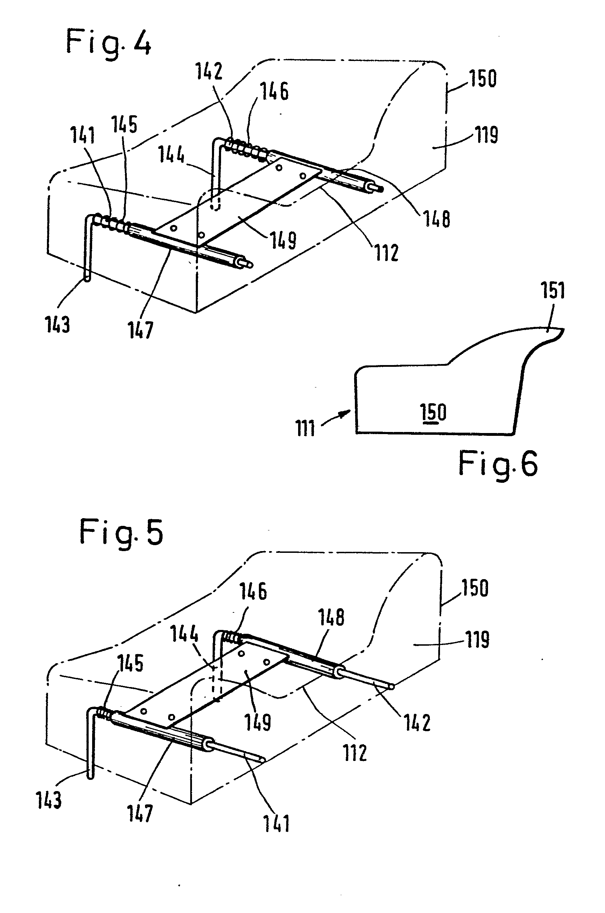

Die Fig. 4 zeigt nun das Rückhaltekissen für die Krankentrage nach der Fig. 3. Dieses Rückhaltekissen weist zwei Kolbenstangen 141, 142 auf, die über ihre nach unten gebogenen Enden 143, 144 in die Öffnungen der Halterung 140 einschiebbar sind. Auf den Kolbenstangen 141, 142 ist jeweils eine Feder 145, 146 vorgesehen. Ausserdem gleitet auf den Kolbenstangen 141, 142 hinter den Federn 145, 146 jeweils ein Kolbengehäuse 147, 148. Die Kolbengehäuse 147, 148 sind durch eine Platte 149 miteinander verbunden. Der Durchmesser der Kolbengehäuse 147, 148 ist derart gewählt, dass durch diese Kolbengehäuse die Feder 145 bzw. 146 zusammengedrückt werden kann, wie dies in Fig. 5 dargestellt ist. Die Kolbenstangen 141, 142, die Federn 145, 146 und die Kolbengehäuse 147, 148 bilden also Stossdämpfer, die in Fig. 4 im entspannten und in Fig. 5 im gespannten Zustand dargestellt sind.

Das Rückhaltekissen 111 enthält eine mit Weichschaum ausgelegte Auswölbung 112 in einem Hartschaum-Formteil 150, das in den Fig. 4 und 5 strichpunktiert dargestellt ist. In diese Auswölbung 112 werden der Kopf und der Hals des Patienten aufgenommen. Wenn sich ein Patient auf der Krankentrage 101 befindet, so liegen dann seine Schultern an einer «harten» Rückhaltefla-che 119 des Rückhaltekissens 111 an, wobei der Gurt 123 über den Oberkörper des Patienten geführt ist.

Das Formteil 150 ist auf einem nicht dargestellten Brett befestigt, an dessen gegebenenfalls verstärkter Unterseite das Gestänge 141, 142 angebracht ist. Ausserdem ist das Formteil 150 mit Kunstleder überzogen, das an der Unterseite des Brettes befestigt ist. Das Brett kann aus Holz oder Kunststoff bestehen.

Fig. 6 zeigt noch eine Seitenansicht des Rückhaltekissens mit einem Polstervorbau 151 der Kissenfüllung 150. Dieser Polstervorbau 151 verhindert, dass der Körper eines Patienten über das Rückhaltekissen 111 rutscht, da wegen der Beweglichkeit des Rückhaltekissens 111 über die Hublänge der Kolbenstangen 141, 142 auf spezielle Sicherheitsgurte für das Rückhaltekissen 111 verzichtet wird.

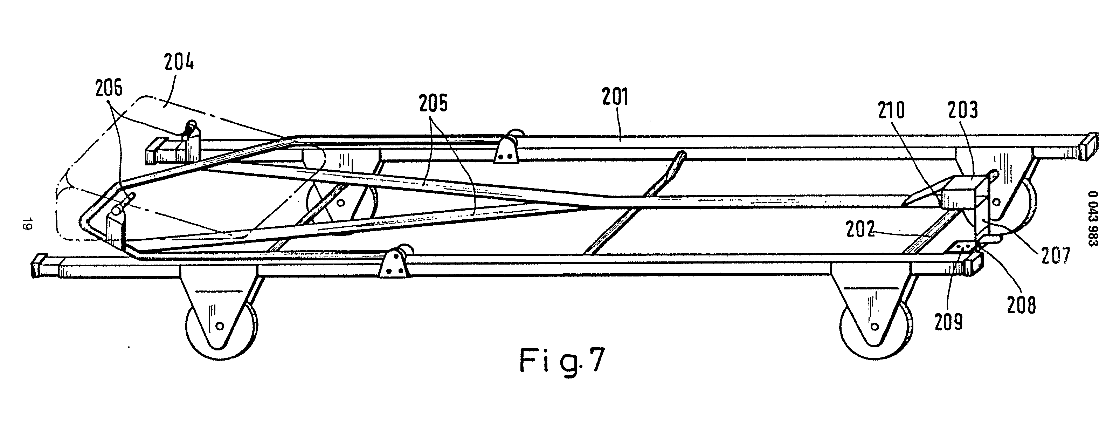

Die Darstellung gemäss Figur 7 zeigt eine schematische perspektivische Ansicht eines weiteren Ausführungsbeispiels einer Krankentrage nach der Erfindung, wobei lediglich das Gestell der Krankentrage 201 veranschaulicht ist und die Liegefläche weggelassen wurde. Bei der in Figur 7 veranschaulichten Ausführungsform ist an einem hinteren Querträger 202 im Bereich des Fussendes der Krankentrage 201 ein Aufrollautomat 203 eines Sicherheitsgurtes 207 um eine Achse 215 schwenkbar gelagert. Der Aufrollautomat 203 ist über einen im vorderen Bereich in Richtung des Kopfendes der Krankentrage 201 in horizontaler Ebene gegabelten Haltegurt 205 mit dem Rückhaltekissen 204 verbunden. Die beiden Schenkel der Gabelung des Haltegurtes 205 sind seitlich an Befestigungen 206 mit dem Rückhaltekissen verankert.

Die Befestigungen 206 sind bevorzugt lösbar ausgebildet, um das Kissen 204 bzw. dessen Bezug leicht auswechseln zu können.

Wie veranschaulicht, verläuft der Haltegurt 205 unterhalb der Liegefläche (nicht dargestellt) der Krankentrage 201.

Der Sicherheitsgurt 207 weist einen Kupplungsteil 208 auf, welcher bei dem veranschaulichten Ausführungsbeispiel als Halteschlaufe ausgebildet ist. Ein entsprechender Kupplungsteil 209 ist am Boden 211 des Krankenfahrzeugs vorgesehen, wobei es sich bei der veranschaulichten Ausführungsform um einen hakenförmigen Vorsprung 209 handelt.

Wie veranschaulicht, ist der Aufrollautomat 203 derart eingestellt, dass die aufgerollte Länge des Sicherheitsgurtes 207 derart begrenzt ist, dass im aufgerollten Zustand die Halteschlaufe 208 sich im geringen Abstand über dem Fahrzeugboden 211 befindet. Beim Einschieben der Krankentrage 201 in die Tragenschienen (nicht dargestellt) im Fahrzeug hängt sich daher die Halteschlaufe 208 selbsttätig in die Halterung 209 ein.

Wie ferner gezeigt, ist der Haltegurt 205 im Bereich des Aufrollautomaten in senkrechter Ebene gegabelt ausgebildet und mit dem Gehäuse des Aufrollautomaten derart verbunden, dass bei Straffung des Haltegurtes 205 die Sperre 210 des Aufrollautomaten betätigt wird.

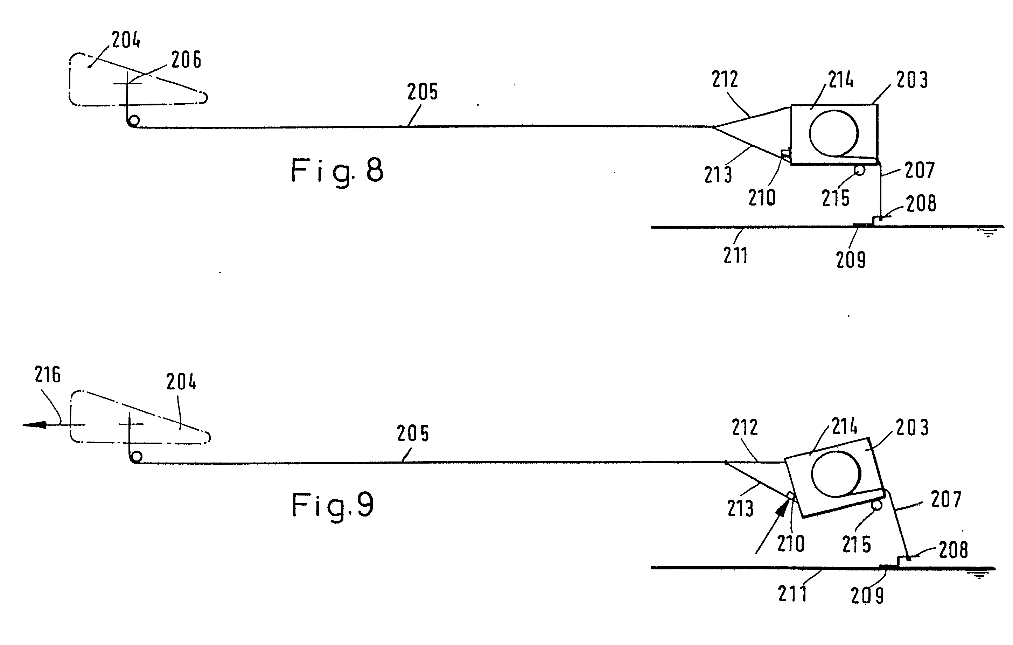

Diese Funktionsweise ist in Figur 8 und 9 dargestellt, wobei in Figur 9 ein Abbremsen des Fahrzeuges durch den Pfeil 216 angedeutet ist.

Bei der in den Zeichnungen veranschaulichten Ausführungsform sind die beiden Abschnitte 212 und 213 der senkrechten Gabelung des Haltegurtes 205 fest mit dem Gehäuse 214 des Aufrollautomaten 203 verbunden, wobei die Anordnung derart getroffen ist (vgl. Figur 9), dass bei einer Bewegung der Krankentrage 201 in Richtung des Pfeiles 216 die daraus resultierende Winkelveränderung zwischen den Abschnitten 212 und 213 die Sperre 210 am Aufrollautomaten 203 sperrt. Da beim Bremsen des Fahrzeuges und beim Überschreiten eines bestimmten Verzögerungswertes der Haltegurt 205 durch die Krankentrage 201 in Richtung des Pfeiles 216 nach vorne gezogen wird, wird hierbei die Sperre 210 durch den unteren Abschnitt 213 nach oben verschwenkt und der Sicherheitsgurt gesperrt.

Bei alternativen Ausführungsformen kann entweder der Haltegurt 205 direkt mit der Sperre 210 oder einer der Abschnitte 212 oder 213 mit der Sperre 210 verbunden werden.

Bei einer weiteren abgewandelten Ausführungsform (in den Zeichnungen nicht dargestellt) ist das Kissen 204 beidseitig verwendbar ausgebildet, wobei bevorzugt die eine Seite des Kissens 204 mit einer Kopfmulde versehen ist, während die gegenüberliegende Seite glatt gepolstert ausgebildet ist.

Abweichend von den veranschaulichten und beschriebenen Ausführungsformen ist es ferner mit Vorteil möglich, die beschriebene Konstruktion eines Rückhaltekissens auch bei Liege-Tragsesseln zu verwenden.

**Patentansprüche**

1. Krankentrage mit einem verstellbaren und arretierbaren Gurt, der als Sicherheitsgurt zur Halterung eines auf der Krankentrage liegenden Patienten dient, dadurch gekennzeichnet, dass an der Krankentrage (1) ein Rückhaltekissen (11, 111) gehalten ist, das über eine Rückhaltefläche (19) an der Schulter des Patienten anliegt, so dass dieser bezüglich der Krankentrage (1) auch bei stossartigen Bewegungsabläufen in deren Längsrichtung (Pfeil I) ortsfest bleibt.

2. Krankentrage nach Anspruch 1, dadurch gekennzeichnet, dass das Rückhaltekissen (11) eine Auswölbung (12) für Kopf und Hals des Patienten enthält.

3. Krankentrage nach Anspruch 1 oder 2, dadurch gekennzeichnet, dass das Rückhaltekissen (11) mit dem Sicherheitsgurt (10) integriert ist.

4. Krankentrage nach Anspruch 3, dadurch gekennzeichnet, dass der Sicherheitsgurt (10) und ein weiterer Sicherheitsgurt (13) über jeweils eine Schulter des Patienten geführt sind.

5. Krankentrage nach Anspruch 4, dadurch gekennzeichnet, dass der Sicherheitsgurt (10) und gegebenenfalls der weitere Sicherheitsgurt (13) mit ihrem einen Ende am Rückhaltekissen (11) und mit ihrem anderen Ende an Seitenholmen (15) der Rückenlehne (26) der Krankentrage (1) befestigt sind.

6. Krankentrage nach Anspruch 5, dadurch gekennzeichnet, dass das andere Ende des Sicherheitsgurtes (10) und gegebenenfalls des weiteren Sicherheitsgurtes (13) an den Seitenholmen (15) in Höhe eines quer zur Längsrichtung (2) der Krankentrage (1) verlaufenden Befestigungsgurtes (23) angebracht sind.

7. Krankentrage nach Anspruch 6, dadurch gekennzeichnet, dass der Sicherheitsgurt (10) und gegebenenfalls der weitere Sicherheitsgurt (13) direkt am Befestigungsgurt (23) angebracht sind.

8. Krankentrage nach einem der Ansprüche 1 bis 7, gekennzeichnet durch wenigstens einen Gurt (16, 17) zum Befestigen des Rückhaltekissens (11) an der Krankentrage (1).

9. Krankentrage nach den Ansprüchen 6 und 8, dadurch gekennzeichnet, dass der Gurt (17) an jeweils einem Seitenholm (15) der Rückenlehne (26) befestigbar ist.

10. Krankentrage nach einem der Ansprüche 3 bis 9, dadurch gekennzeichnet, dass der Sicherheitsgurt (10) und gegebenenfalls der weitere Sicherheitsgurt (13) über ein Automatikgehäuse (18) am Rückhaltekissen (11) angebracht sind.

11. Krankentrage nach einem der Ansprüche 3 bis 10, dadurch gekennzeichnet, dass die Sicherheitsgurte (10, 13) in ihrer Länge verstellbar sind.

12. Krankentrage nach Anspruch 8, dadurch gekennzeichnet, dass der Gurt (16, 17) in seiner Länge verstellbar ist.

13. Krankentrage nach Anspruch 1 oder 2, dadurch gekennzeichnet, dass das Rückhaltekissen (111) elastisch gelagert ist, so dass ein abruptes Abfangen des Patienten bei den stossartigen Bewegungsabläufen vermieden wird.

14. Krankentrage nach Anspruch 13, dadurch gekennzeichnet, dass das Rückhaltekissen (111) durch wenigstens eine Feder (145, 146) elastisch gelagert ist.

15. Krankentrage nach Anspruch 14, dadurch gekennzeichnet, dass die Feder (145, 146) auf einem Gestänge (141, 142) gelagert ist.

16. Krankentrage nach Anspruch 15, dadurch gekennzeichnet, dass das Gestänge (141, 142) mit der Feder (145, 146) in das Rückhaltekissen (111) integriert ist.

17. Krankentrage nach Anspruch 15 oder 16, dadurch gekennzeichnet, dass das Gestänge (141, 142) vorzugsweise am Kopfende (140) der Krankentrage (101) verankerbar ist.

18. Krankentrage nach einem der Ansprüche 15 bis 17, dadurch gekennzeichnet, dass das Gestänge zwei in Längsrichtung der Krankentrage (101) verlaufende Kolbenstangen (141, 142) aufweist, auf denen jeweils ein Kolbengehäuse (147, 148) mit einer Feder (145, 146) gleitet.

19. Krankentrage nach Anspruch 18, dadurch gekennzeichnet, dass die beiden Kolbengehäuse (147, 148) starr miteinander verbunden sind.

20. Krankentrage nach Anspruch 19, dadurch gekennzeichnet, dass die beiden Kolbengehäuse (147, 148) durch eine Platte (149) miteinander verbunden sind.

21. Krankentrage nach einem der Ansprüche 15 bis 17, dadurch gekennzeichnet, dass das Gestänge (141, 142) an der Unterseite einer insbesondere aus Holz bestehenden Platte befestigt ist, auf der sich ein Hartschaum-Formteil (150) befindet.

22. Krankentrage nach Anspruch 21, dadurch gekennzeichnet, dass das Hartschaum-Formteil (150) im Kopfbereich (112) mit Weichschaum belegt ist.

23. Krankentrage nach Anspruch 21 oder 22, dadurch gekennzeichnet, dass das Hartschaum-Formteil (150) und die Weichschaum-Belegung mit einem Kunstlederüberzug versehen sind.

24. Krankentrage nach einem der Ansprüche 13 bis 23, dadurch gekennzeichnet, dass das Rückhaltekissen (111) einen Polstervorbau (151) aufweist.

25. Krankentrage nach Anspruch 1, dadurch gekennzeichnet, dass das Rückhaltekissen (204) an einem Haltegurt (205) befestigt ist, dass ein Sicherheitsgurt (207) mit Aufrollautomatik (203) und einem Kupplungsteil (208) vorgesehen ist, dass am Fahrzeugboden (211) ein entsprechendes zweites Kupplungsteil (209) vorgesehen ist, in welchen sich der Kupplungsteil (208) des Sicherheitsgurtes (207) beim Einladen der Krankentrage (201) in eine Trageneinrichtung selbsttätig einhängt und dass der Haltegurt (205) mit der Aufrollautomatik (203) derart verbunden ist, dass eine Zugkraft in dem Haltegurt (205) die Aufrollautomatik (203) sperrt.

26. Krankentrage nach Anspruch 25, dadurch gekennzeichnet, dass die Aufrollautomatik (203) an einem Querträger (202) der Krankentrage (201) im Bereich des Fussendes beweglich angeordnet ist.

27. Krankentrage nach Anspruch 25 oder 26, dadurch gekennzeichnet, dass der Haltegurt (205) unterhalb der Liegefläche der Krankentrage (201) verlaufend angeordnet ist.

28. Krankentrage nach einem der Ansprüche 25 bis 27, dadurch gekennzeichnet, dass der Haltegurt (205) im Bereich des Kopfendes der Krankentrage (201) seitlich gegabelt ausgebildet ist und an beiden Seiten des Rückhaltekissens (204) Befestigungen (206) aufweist.

29. Krankentrage nach Anspruch 28, dadurch gekennzeichnet, dass die Befestigungen (206) lösbar ausgebildet sind.

30. Krankentrage nach einem der vorstehenden Ansprüche, dadurch gekennzeichnet, dass das Rückhaltekissen (204) beidseitig verwendbar ausgebildet ist.

31. Krankentrage nach Anspruch 30, dadurch gekennzeichnet, dass die eine Seite des Rückhaltekissens (204) mit einer Kopfmulde versehen ist und die gegenüberliegende Seite glatt gepolstert ausgebildet ist.

32. Krankentrage nach einem der Ansprüche 25 bis 31, dadurch gekennzeichnet, dass der Kupplungsteil (208) des Sicherheitsgurtes (207) als Halteschlaufe (208) ausgebildet ist und dass der Kupplungsteil (209) am Fahrzeugboden (211) als Haken (209) od. dgl. ausgebildet ist, in welchen sich die Halteschlaufe (208) selbsttätig einhängt.

33. Krankentrage nach Anspruch 32, dadurch gekennzeichnet, dass die durch die Aufrollautomatik (203) aufgerollte Länge des Sicherheitsgurtes (207) derart begrenzt ist, dass im eingerollten Zustand die Halteschlaufe (208) sich im geringen Abstand über dem Fahrzeugboden (211) befindet.

34. Krankentrage nach einem der Ansprüche 25 bis 33, dadurch gekennzeichnet, dass der Haltegurt (205) direkt mit der Sperre (210) der Aufrollautomatik (203) verbunden ist.

35. Krankentrage nach einem der Ansprüche 25 bis 34, dadurch gekennzeichnet, dass der Haltegurt (205) im Bereich des Fussendes der Krankentrage (201) nach oben und unten gegabelt ausgebildet ist, wobei ein Abschnitt (213) der Gabelung mit der Sperre (210) verbunden ist und der andere Abschnitt (212) die Zugkräfte aufnimmt.

36. Krankentrage nach einem der Ansprüche 25 bis 35, dadurch gekennzeichnet, dass der Haltegurt (205) im Bereich des Fussendes der Krankentrage (201) nach oben und unten gegabelt ausgebildet ist, dass die beiden Abschnitte (212, 213) der Gabelung mit dem Gehäuse (214) der Aufrollautomatik (203) fest verbunden sind und dass einer der Abschnitte (212, 213) derart angeordnet ist, dass eine Winkelveränderung zwischen den Abschnitten (212, 213) die Sperre (210) der Aufrollautomatik auslöst.

37. Rückhaltekissen für eine Krankentrage, das über eine Rückhaltefläche an der Schulter eines auf der Krankentrage liegenden Patienten anliegt, wobei im Rückhaltekissen (111) elastische Mittel (145, 146) integriert sind, durch die das Rückhaltekissen (111) bezüglich der Krankentrage (101) elastisch lagerbar ist.

**Claims**

1. A stretcher comprising a belt, which is adjustable and adapted to be locked and serves as a safety belt for retaining a patient lying on the stretcher, characterized in that a restraining pad (11, 111) is held on the stretcher (1) and has a restraining surface (19), which contacts the shoulder of the patient so that the patient will be held in position relative to the stretcher (1) even in case of sudden movements in its longitudinal direction (arrow I).

2. A stretcher according to claim 1, characterized in that the restraining pad (11) has a concave recess (12) for the head and neck of the patient.

3. A stretcher according to claim 1 or 2, characterized in that the restraining pad (11) is integrated with the safety belt (19).

4. A stretcher according to claim 3, characterized in that the safety belt (10) and a further safety belt (13) are laid over respective shoulders of the patient.

5. A stretcher according to claim 4, characterized in that the safety belt (10) and optionally the further safety belt (13) is secured at one end to the restraining pad (11) and at the other end to side members (15) of the backrest (26) of the stretcher (1).

6. A stretcher according to claim 5, characterized in that the safety belt (10) and optionally the further safety belt (13) is attached at its other end to the side members (15) on the level of a fixing strap (23), which extends transversely to the longitudinal direction (2) of the stretcher (1).

7. A stretcher according to claim 6, characterized in that the safety belt (10) and optionally the further safety belt (13) are directly attached to the fixing strap (23).

8. A stretcher according to any of claims 1 to 7, characterized by at least one strap (16, 17) for fixing the restraining pad (11) to the stretcher (1).

9. A stretcher according to claims 6 and 8, characterized in that the strap (17) is adapted to be secured to both side members (15) of the backrest (26).

10. A stretcher according to any of claims 3 to 9, characterized in that the safety belt (10) and optionally the further safety belt (13) are attached to the restraining pad (11) by an automatic retractor housing (18).

11. A stretcher according to any of claims 3 to 10, characterized in that the safety belts (10, 13) are adjustable in length.

12. A stretcher according to claim 8, characterized in that the strap (16, 17) is adjustable in length.

13. A stretcher according to claim 1 or 2, characterized in that the restraining pad (111) is elastically mounted so that an abrupt impact of the patient in case of sudden movements will be avoided.

14. A stretcher according to claim 13, characterized in that the restraining pad (111) is elastically mounted by means of at least one spring (145, 146).

15. A stretcher according to claim 14, characterized in that the spring (145, 146) is mounted on rod means (141, 142).

16. A stretcher according to claim 15, characterized in that the rod means (141, 142) and the spring (145, 146) are integrated in the restraining pad (111).

17. A stretcher according to claim 15 or 16, characterized in that the rod means (141, 142) are adapted to be anchored preferably at the head end (140) of the stretcher (101).

18. A stretcher according to any of claims 15 to 17, characterized in that the rod means comprise two piston rods (141, 142), which extend in the longitudinal direction of the stretcher (101), and a piston housing (147, 148) provided with a spring (145, 146) is slidable on each of said piston rods.

19. A stretcher according to claim 18, characterized in that the two piston housings (147, 148) are rigidly interconnected.

20. A stretcher according to claim 19, characterized in that the two piston housings (147, 148) are interconnected by a plate (149).

21. A stretcher according to any of claims 15 to 17, characterized in that the rod means (141, 142) are secured to the underside of a plate, which consists particularly of wood and is provided with a hard foam moulding (150).

22. A stretcher according to claim 21, characterized in that the hard foam moulding (150) is covered with soft foam in the head region (112).

23. A stretcher according to claim 21 or 22, characterized in that the hard foam moulding (150) and the soft foam covering are provided with a covering of artificial leather.

24. A stretcher according to any of claims 13 to 23, characterized in that the restraining pad (111) comprises a cushioning forward extension (151).

25. A stretcher according to claim 1, characterized in that the restraining pad (204) is secured to a retaining strap (205), that a safety belt (207) provided with an automatic retractor (203) and with a coupling member (208) is provided, a corresponding second coupling member (209) is provided on the floor (211) of the vehicle, the coupling member (208) of the safety belt (207) is automatically received by the second coupling member (209) as the stretcher (201) is loaded into carrying means, and the retaining strap (205) is connected to the automatic retractor (203) in such a manner that the automatic retractor (203) will be blocked in response to a tensile force exerted by the retaining belt (205).

26. A stretcher according to claim 25, characterized in that the automatic retractor (203) is movably mounted on a cross-member (202) of the stretcher adjacent to the foot end thereof.

27. A stretcher according to claim 25 or 26, characterized in that the retaining strap (205) extends below the patient-supporting surface of the stretcher (201).

28. A stretcher according to any of claims 25 to 27, characterized in that the retaining strap (205) is laterally forked adjacent to the head end of the stretcher (201) and is provided with fixing means (206) on both sides of the restraining pad (204).

29. A stretcher according to claim 28, characterized in that the fixing means (206) are detachable.

30. A stretcher according to any of the preceding claims, characterized in that the restraining pad (204) is designed to be used on both sides.

31. A stretcher according to claim 30, characterized in that the restraining pad (204) is provided on one side with a depression for the head and has on the other side a smooth upholstered surface.

32. A stretcher according to any of claims 25 to 31, characterized in that the coupling member (208) of the safety belt (207) consists of a retaining loop (208) and the coupling member (209) on the floor (211) of the vehicle consists of a hook (209) or the like for automatically receiving the retaining loop (208).

33. A stretcher according to claim 32, characterized in that the automatic retractor (203) is adapted to retract the safety belt (207) only in such a limited length that the retaining loop (208) is closely spaced over the floor (211) of the vehicle when the safety belt (207) has been retracted.

34. A stretcher according to any of claims 25 to 33, characterized in that the retaining strap (205) is directly connected to the locking means (210) of the automatic retractor (203).

35. A stretcher according to any of claims 25 to 34, characterized in that the retaining strap (205) is forked upwardly and downwardly adjacent to the foot end of the stretcher (201), one leg (213) of the forked portion is connected to the locking means (210) and the other leg (212) takes up the tensile forces.

36. A stretcher according to any of claims 25 to 35, characterized in that the retaining strap (205) is forked upwardly and downwardly adjacent to the foot end of the stretcher (201), the two legs (212, 213) of the forked portion are fixedly connected to the housing (214) of the automatic retractor (203) and one of the legs (212, 213) is so arranged that a change of the angle between the legs (212, 213) will release the locking means (210) of the automatic retractor.

37. A restraining pad for a stretcher, which pad has a restraining surface in contact with the shoulder of a patient lying on the stretcher, wherein elastic means (145, 146) are integrated in the restraining pad (111) and are adapted to elastically mount the restraining pad (111) relative to the stretcher (101).

**Revendications**

1. Brancard muni d'une ceinture pouvant être réglée et bloquée, qui sert de ceinture de sécurité pour maintenir un malade allongé, sur le brancard caractérisé en ce que sur le brancard (1) est fixé un coussin de retenue (11, 111) qui appuie par une surface de retenue (19) contre l'épaule du malade, de sorte que celui-ci reste au même endroit par rapport au brancard (1) même dans le cas de mouvements brusques dans le sens longitudinal de celui-ci (flèche 1).

2. Brancard selon la revendication 1, caractérisé en ce que le coussin de retenue (11) comporte une partie en creux (12) pour la tête et le cou du malade.

3. Brancard selon la revendication 1 ou 2, caractérisé en ce que le coussin de retenue (11) est intégré avec la ceinture de sécurité (10).

4. Brancard selon la revendication 3, caractérisé en ce qu'une première ceinture de sécurité (10) et une deuxième ceinture de sécurité (13) passent chacune sur une épaule respective du malade.

5. Brancard selon la revendication 4, caractérisé en ce que la première ceinture de sécurité (10) et éventuellement la deuxième ceinture de sécurité (13) sont fixées par l'une de leurs extrémités au coussin de retenue (11) et par leur autre extrémité à des longerons latéraux (15) du dossier (26) du brancard (1).

6. Brancard selon la revendication 5, caractérisé en ce que la seconde extrémité de la première ceinture de sécurité (10) et éventuellement celle de la deuxième ceinture de sécurité (13) sont placées sur les longerons (15) au niveau d'une ceinture de fixation (23) perpendiculaire à la direction longitudinale (2) du brancard (1).

7. Brancard selon la revendication 6, caractérisé en ce que la première ceinture de sécurité (10) et éventuellement la deuxième ceinture de sécurité (13) sont placées directement sur la ceinture de fixation (23).

8. Brancard selon l'une quelconque des revendications 1 à 7, caractérisé en ce qu'il comporte au moins une courroie (16, 17) pour fixer le coussin de retenue (11) sur le brancard-courroie (16, 17) pour fixer le coussin de retenue (11) sur le brancard (1).

9. Brancard selon les revendications 6 et 8, caractérisé en ce que la courroie (17) peut être fixée sur un longeron latéral respectif (15) du dossier (26).

10. Brancard selon l'une des revendications 3 à 9, caractérisé en ce que la première ceinture de sécurité (10) et éventuellement la deuxième ceinture de sécurité (13) sont placées sur le coussin de retenue (11) par l'intermédiaire d'un boîtier d'enrouleur automatique (18).

11. Brancard selon l'une des revendications 3 à 10, caractérisé en ce que les ceintures de sécurité (10, 13) sont réglables en longueur.

12. Brancard selon la revendication 8, caractérisé en ce que la courroie (16, 17) est réglable en longueur.

13. Brancard selon la revendication 1 ou 2, caractérisé en ce que le coussin de retenue (11) est suspendu élastiquement, ce qui évite que le patient soit sollicité brutalement lors de mouvements brusques.

14. Brancard selon la revendication 13, caractérisé en ce que le coussin de retenue (11) est suspendu élastiquement au moyen d'au moins un ressort (145, 146).

15. Brancard selon la revendication 14, caractérisé en ce que le ressort (145, 146) est placé sur une tringle (141, 142).

16. Brancard selon la revendication 15, caractérisé en ce que la tringle (141, 142) est intégrée avec le ressort (145, 146) dans le coussin de retenue (111).

17. Brancard selon la revendication 15 ou 16, caractérisé en ce que la tringle (141, 142) peut être ancrée de préférence à l'extrémité de tête (140) du brancard (101).

18. Brancard selon l'une quelconque des revendications 15 à 17, caractérisé en ce que la tringle est constituée par deux tiges de piston (141, 142) dirigées dans la direction longitudinale du brancard (101), sur chacune desquelles coulisse un boîtier de piston respectif (147, 148) comportant un ressort (145, 146).

19. Brancard selon la revendication 18, caractérisé en ce que les deux boîtiers de piston (147, 148) sont reliés rigidement entre eux.

20. Brancard selon la revendication 19, caractérisé en ce que les deux boîtiers de piston (147, 148) sont reliés entre eux par une plaque (149).

21. Brancard selon l'une des revendications 15 à 17, caractérisé en ce que la tringle (141, 142) est fixée sur le dessous d'une plaque, en particulier en bois, sur laquelle se trouve un préformé de mousse rigide (150).

22. Brancard selon la revendication 21, caractérisé en ce que le préformé de mousse rigide (150) est garni de mousse souple dans la zone de tête (112).

23. Brancard selon la revendication 21 ou 22, caractérisé en ce que le préformé de mousse rigide (150) et la garniture de mousse souple comportent un revêtement de cuir synthétique.

24. Brancard selon l'une des revendications 13 à 23, caractérisé en ce que le coussin de retenue (111) comporte un avant-corps rembourré (151).

25. Brancard selon la revendication 1, caractérisé en ce que le coussin de retenue (204) est fixé sur une courroie de retenue (205), qu'on prévoit une ceinture de sécurité (207) munie d'un enrouleur automatique (203) et d'un élément d'accouplement (208), qu'on prévoit sur le plancher du véhicule (211) un second élément d'accouplement correspondant (209) dans lequel s'accroche automatiquement l'élément d'accouplement (208) de la ceinture de sécurité (207) lorsqu'on charge le brancard (201) dans un dispositif support et que la courroie de retenue (205) est reliée à l'enrouleur automatique (203) de telle sorte qu'une force de traction dans la courroie de retenue (205) bloque l'enrouleur automatique (203).

26. Brancard selon la revendication 25, caractérisé en ce que l'enrouleur automatique (203) est disposé de façon à être mobile sur une traverse (202) du brancard (201) dans la zone de l'extrémité de pied.

27. Brancard selon la revendication 25 ou 26, caractérisé en ce que la ceinture de retenue (205) est placée et s'étend sous la surface de repos du brancard (201).

28. Brancard selon l'une des revendications 25 à 27, caractérisé en ce que la ceinture de retenue (205) bifurque vers les côtés dans la zone de l'extrémité de tête du brancard (201) et comporte des fixations (206) de chaque côté du coussin de retenue (204).

29. Brancard selon la revendication 28, caractérisé en ce que les fixations (206) sont réalisées de façon à être amovibles.

30. Brancard selon l'une des revendications précédentes, caractérisé en ce que le coussin (204) est conformé de façon à pouvoir être utilisé des deux côtés.

31. Brancard selon la revendication 30, caractérisé en ce que l'un des côtés du coussin de retenue (204) comporte un creux pour la tête, tandis que le côté opposé a la forme d'un rembourrage lisse.

32. Brancard selon l'une des revendications 25 à 31, caractérisé en ce que la pièce d'accouplement (208) de la ceinture de sécurité (207) a la forme d'un étrier de retenue (208), et en ce que la pièce d'accouplement (209) située sur le plancher (211) a la forme d'un crochet (209) ou l'analogue dans lequel l'étrier de retenue (208) s'accroche automatiquement.

33. Brancard selon la revendication 32, caractérisé en ce que la longueur de la ceinture de sécurité (207) qu'enroule l'enrouleur automatique (203) est limitée de façon que dans l'état enroulé, l'étrier de retenue (208) se trouve à une faible distance au-dessus du plancher (211) du véhicule.

34. Brancard selon l'une des revendications 25 à 33, caractérisé en ce que la courroie de retenue (205) est reliée directement au dispositif de blocage (210) de l'enrouleur automatique (203).

35. Brancard selon l'une des revendications 25 à 34, caractérisé en ce que la ceinture de retenue (205) bifurque vers le haut et vers le bas au voisinage de l'extrémité de pied du brancard (201), une branche (213) de cette bifurcation étant repliée au dispositif de blocage (210), tandis que l'autre branche (212) encaisse les forces de traction.

36. Brancard selon l'une des revendications 25 à 35, caractérisée en ce que la ceinture de retenue (205) bifurque vers le haut et vers le bas dans la zone de l'extrémité de pied de la civière (201), en ce que les deux branches (212, 213) de cette bifurcation sont reliées rigidement au boîtier (214) de l'enrouleur automatique (203) et en ce que l'une des branches (212, 213) est disposée de façon qu'une modification de l'angle entre les branches (212, 213) déclenche le dispositif de blocage (210) de l'enrouleur automatique.

37. Coussin de retenue pour un brancard qui appuie par l'intermédiaire d'une surface de retenue contre l'épaule d'un malade allongé sur le brancard, des moyens élastiques (145, 146) étant intégrés dans le coussin de retenue (111), lesquels permettent de monter le coussin de retenue (111) élastiquement par rapport au brancard (101).

Fig.1

Fig. 2

0 043 983

Fig.3

Fig. 4

Fig. 6

Fig. 5

Fig. 7

0 043 983

Fig. 8

Fig. 9